# EUROPÄISCHE PATENTANMELDUNG

(11) **EP 3 875 020 A1**
(43) Veröffentlichungstag der Anmeldung: **08.09.2021**
(21) Anmeldenummer: 21158031.1
(22) Anmeldetag: 19.02.2021
(51) Int. Cl.: A61B 1/00, A61B 1/07, B33Y 80/00, A61B 1/002, A61B 1/055

(54) **ENDOSKOP MIT EINER LICHTLEITEREINRICHTUNG, VERFAHREN ZUR HERSTELLUNG EINES ENDOSKOPS MIT EINER LICHTLEITEREINRICHTUNG UND VERWENDUNG EINES VERFAHRENS ZUR HERSTELLUNG EINES ENDOSKOPS**

(30) Priorität: 02.03.2020 DE 102020105469
(71) Anmelder: Karl Storz SE & Co. KG, 78532 Tuttlingen (DE)
(72) Erfinder: EISENLAUER, Johannes, 78532 Tuttlingen (DE)
(74) Vertreter: Patent- und Rechtsanwälte Behrmann Wagner PartG mbB

(57) **Zusammenfassung**

1. Die Erfindung betrifft ein Verfahren zur Herstellung eines Endoskops (02) mit einer Lichtleitereinrichtung umfassend die Verfahrensschritte:
- Bereitstellen eines Innenschaftes (03) zur Aufnahme einer Abbildungsoptik (S1);
- Anordnen eines Führungssegmentes (09) zur Ausrichtung des Innenschaftes (03) in einem Außenschaft (04) und/oder zur Führung von Lichtleitelementen (06) der Lichtleitereinrichtung auf der Außenoberfläche (11) des Innenschaftes (03), insbesondere in einem distalen Endabschnitt (01) des Außenschaftes (04);
- Anordnen der Lichtleitelemente (06) auf der Außenoberfläche (11) des Innenschaftes (03; S3);
- Einführen des Innenschaftes (03) samt Lichtleitelementen (06) in den Außenschaft (04) unter Anlage einer radial äußeren Oberfläche (12) des Führungssegments (09) an einer Innenoberfläche (13) des Außenschafts (04; S4);
- Befestigen des Innenschaftes (03) und der Lichtleitelemente (06) im Außenschaft (04), zumindest im distalen Endabschnitt (01) des Innenschaftes (03) und Außenschaftes (04; S5), wobei vorgesehen ist, dass das Führungssegment (09) durch einen additiven Fertigungsprozess unmittelbar auf der Außenoberfläche (11) des Innenschaftes (03) ausgebildet wird (S2).

## Beschreibung

Die vorliegende Erfindung betrifft ein Verfahren zum Herstellen eines Endoskops mit einer Lichtleitereinrichtung gemäß dem Oberbegriff des Anspruchs 1. Ferner betrifft die vorliegende Erfindung eine Verwendung eines Verfahrens zur Herstellung eines Endoskops gemäß dem Oberbegriff des Anspruchs 5. Außerdem betrifft die vorliegende Erfindung ein Endoskop mit einer Lichtleitereinrichtung gemäß dem Oberbegriff des Anspruchs 6.

In der medizinischen und technischen Endoskopie ist in der Regel eine Beleuchtung des betrachteten Objekts erforderlich. Zur Erzeugung von Beleuchtungslicht mit einem hohen Lichtstrom werden oft separate oder in das proximale Ende des Endoskops integrierte Lichtquelleneinrichtungen verwendet. Vom proximalen Ende zum distalen Ende des Endoskops wird das Beleuchtungslicht mittels eines oder mehrerer Bündel von Lichtleiterelementen einer Lichtleitereinrichtung übertragen.

Im Stand der Technik ist es dabei bekannt, die Lichtleitelemente zwischen einem Außenschaft und einem Innenschaft des Endoskops zum distalen Ende zu führen, wobei der Außenschaft mit seiner Außenoberfläche die äußere Begrenzung des Endoskops mit ausbildet und der Innenschaft zur Aufnahme einer Abbildungsoptik vorgesehen ist.

Um eine gewünschte Anordnung des Innenschaftes gegenüber dem Außenschaft zu erreichen oder zu ermöglichen und gleichzeitig eine gewünschte Anordnung und Ausrichtung der Lichtleitelemente im distalen Endabschnitt des Endoskops in seinem fertig oder endgültig montierten Zustand zu ermöglichen, sind aus dem Stand der Technik bereits Führungssegmente oder allgemein Segmente bekannt, die auf der Außenoberfläche des Innenschaftes im distalen Endabschnitt des Innenschaftes angeordnet werden und durch ihre Form in radialer Richtung oder in Umfangsrichtung, insbesondere durch eine variable Höhe oder Dicke in radialer Richtung die Ausrichtung des Innenschaftes gegenüber dem Außenschaft bestimmen, wobei die Erstreckung oder winkelmäßige Ausdehnung des Segments oder Führungssegments in Umfangsrichtung die Position oder Anordnung der Lichtleitelemente im distalen Endabschnitt des Endoskops mitbestimmt und die Form des Segments oder Führungssegments in axialer Richtung, insbesondere an den in Umfangsrichtung liegenden, sich radial erstreckenden Rändern, die Ausrichtung der Lichtleitelemente mitbestimmt.

Somit kann zusätzlich zur Relativanordnung zwischen Außenschaft und Innenschaft über die Form des Segments oder Führungssegments die Anordnung und Ausrichtung der Lichtleitelemente im distalen Endabschnitt des Endoskops bestimmt werden, sodass beispielsweise eine gewünschte Ausrichtung, bevorzugt gewinkelt zur axialen Erstreckung des Innen- und/oder Außenschaftes, bei einer gleichzeitig gebündelten oder konzentrierten Anordnung der Lichtleitelemente in einer gleichmäßigen Art und Weise erreicht werden kann.

Im Stand der Technik ist zur Erzeugung und/oder Anordnung des Segments oder Führungssegments auf dem Innenschaft in der Regel ein mehrstufiger Prozess oder ein mehrstufiges Verfahren vorgesehen. Dabei wird zunächst einen Rohling oder Grundkörper/Grünkörper des Segments oder Führungssegments im distalen Endabschnitt des Innenschaftes aufgelötet. Diese Vorstufe des endgültigen Führungssegments im Hinblick auf die äußere Form weist in der Regel noch eine gleichmäßige Höhe, Stärke oder radiale Erstreckung auf. Um eine oftmals benötigte oder gewünschte, in Umfangsrichtung variable Höhe oder Stärke des Führungssegments zu erreichen, wird die Baugruppe umfassend den Innenschaft und die Vorstufe des Führungssegments anschließend in einem spanenden Bearbeitungsschritt, bevorzugt durch ein Überdrehen, so bearbeitet, dass die gewünschte Form des Führungssegments, insbesondere die gewünschte in Umfangsrichtung variable Stärke oder Höhe des Führungssegments erreicht wird.

Allgemein nachteilig an dieser Art der Herstellung oder Bereitstellung der Baugruppe aus Innenschaft und Führungssegment ist einerseits die verhältnismäßig hohe Anzahl an Bearbeitungsschritten. Auch wird im Stand der Technik als Material des Führungselements oftmals Messing eingesetzt, da es sich beim Löten und Überdrehen gut verarbeiten lässt. Die an sich geringe Biokompatibilität und/oder die Einstufung von Messing als Allergen sind jedoch nur tolerierbar, da auf Grund der geringen Fläche oder Oberfläche der Führungselemente die potentiell auslösbaren biochemischen Reaktionen gering sind. Ein weiterer Nachteil besteht insbesondere auch in der bisher bekannten Vorgehensweise zur Befestigung des Rohlings des Führungssegments oder des Verbindens des Innenschaftes mit dem Rohling des Führungssegments. Denn durch das Auflöten unter Verwendung eines Lots als Verbindungsmaterial, können insbesondere bei einem nachfolgenden oder anschließenden mechanischen, bevorzugt spanenden, Bearbeitungsprozess Probleme mit der Haftung auftreten, die im ungünstigsten Fall dazu führen, dass das aufgelötete Führungssegment oder dessen Vorstufe, vom Innenschaft abgesprengt werden oder abplatzen.

Der letztgenannte Nachteil tritt besonders bei Endoskopen mit kleinem Durchmesser, insbesondere kleinem Durchmesser des Außenschaftes auf. Bei derartigen Endoskopen kann durch ein Auflöten eines Segments oder einer Vorstufe eines Führungssegments keine ausreichende Haftwirkung erzeugt werden, sodass bei einem nachfolgenden spanenden Bearbeitungsprozess, insbesondere bei einem Überdrehen, die Haftung des Führungssegments am Innenschaft oder auf der Außenoberfläche des Innenschaftes nicht mit ausreichender Reproduzierbarkeit gewährleistet ist.

Diese Problematiken haben insgesamt zu dem Umstand geführt, dass im Stand der Technik für Endoskope mit entsprechend kleinem Durchmesser, insbesondere mit einem Durchmesser des Außenschaftes von ≤4mm, keine entsprechenden Führungssegmente zum Einsatz kommen. Dies wiederum führt jedoch zu besonderen Herausforderungen im Fertigungsprozess der bekannten Endoskope mit geringem Durchmesser. Denn die Anordnung des Innenschaftes gegenüber dem Außenschaft sowie die Anordnung und Ausrichtung der Lichtleitelemente, ganz besonders im distalen Endabschnitt des Endoskops, hängen ohne die Verwendung und die Wirkung eines wie oben beschriebenen Führungssegments ganz wesentlich von der Fertigkeit, insbesondere Fingerfertigkeit des jeweiligen Montagearbeiters ab. Dies führt jedoch dazu, dass nur mit entsprechend hohem Zeitaufwand, unter Einsatz entsprechend versierter Fertigungskräfte und gleichzeitig unter Inkaufnahme nicht unerheblicher Mengen an Ausschuss die bekannten Endoskope mit kleinem Durchmesser produziert werden können.

Der vorliegenden Erfindung liegt daher die Aufgabe zu Grunde die vorangehend genannten Nachteile im Stand der Technik zu überwinden und insbesondere ein Endoskop und ein Verfahren zur Herstellung eines Endoskops, sowie eine Verwendung des Verfahrens vorzuschlagen, die eine schnelle, sichere und zuverlässige Anordnung eines bekannten Führungssegments auf der Außenoberfläche des Innenschaftes des Endoskops erlauben.

Im Hinblick auf das erfindungsgemäße Verfahren wird die oben genannte Aufgabe mit den Merkmalen des Anspruchs 1 gelöst. Bezüglich der Verwendung des Verfahrens wird die erfindungsgemäße Aufgabe mit den Merkmalen des Anspruchs 5 gelöst. Im Hinblick auf ein erfindungsgemäßes Endoskop wird die Aufgabe mit den Merkmalen des Anspruchs 6 gelöst. Bevorzugte Ausführungsformen sind jeweils Gegenstand der Unteransprüche.

Das erfindungsgemäße Verfahren zur Herstellung eines Endoskops mit einer Lichtleitereinrichtung sieht zunächst folgende an sich bekannte Verfahrensschritte vor:
- Bereitstellen eines Innenschaftes zur Aufnahme einer Abbildungsoptik;
- Anordnen eines Führungssegments zur Ausrichtung des Innenschaftes in einem Außenschaft und/oder zur Führung von Lichtleitelementen der Lichtleitereinrichtung auf der Außenoberfläche des Innenschaftes, insbesondere in einem distalen Endabschnitt des Innenschaftes;
- Anordnen der Lichtleitelemente auf der Außenoberfläche des Innenschaftes;
- Einführen des Innenschaftes samt Lichtleitelementen in den Außenschaft unter Anlage einer radial äußeren Oberfläche des Führungssegments an einer Innenoberfläche des Außenschaftes.
- Befestigen des Innenschaftes und der Lichtleitelemente im Außenschaft, zumindest im distalen Endabschnitt des Innenschaftes und Außenschaftes.

Erfindungsgemäß ist dabei jedoch vorgesehen, dass das Führungssegment durch einen additiven Fertigungsprozess unmittelbar auf der Außenoberfläche des Innenschaftes ausgebildet wird. Dies bedeutet mit anderen Worten ausgedrückt, dass insbesondere kein Lot oder sonstiges Verbindungsmaterial abgesehen von dem Material des Innenschaftes und dem Material des Führungssegments bei der Befestigung oder Verbindung zum Einsatz kommt. Wie später noch ausführlicher dargelegt werden wird, ist es zwar möglich, dass sich in einem Übergangsbereich zwischen dem Material des Innenschaftes und dem Material des Führungssegments ein Mischmaterial oder eine Übergangszone ausbildet, die dann beide Materialien und/oder Mischungen der beiden Materialien aufweist, es wird jedoch bei der unmittelbaren Aufbringung des Führungssegments auf dem Innenschaft kein weiteres Material, wie beispielsweise Lot, verwendet.

Die Grundidee der vorliegenden Erfindung basiert also auf dem Verbinden des Führungssegments mit dem Material des Führungssegments unmittelbar auf der Außenoberfläche des Innenschaftes, wozu ein entsprechender additiver Fertigungsprozess verwendet wird. Dadurch kann die Haftung des Führungssegments auf dem Innenschaft deutlich verbessert werden. Gleichzeitig können Prozessschritte vereinfacht werden oder sogar wegfallen, da das Führungssegment unmittelbar auf der Außenoberfläche des Innenschafts ausgebildet wird ohne das Befestigungsmaterial oder Mittlerschichten eines weiteren Materials erzeugt und/oder angebracht oder aufgebracht werden müssen. Durch die unmittelbare oder direkte Aufbringung und Ausbildung des Führungssegments auf dem Innenschaft, insbesondere auf dessen Außenoberfläche wird zudem eine besonders langlebige Verbindung zwischen Innenschaft und Führungssegment erzeugt.

Gemäß einer ersten vorteilhaften Ausführungsform des Verfahrens kann vorgesehen sein, dass das Führungssegment in seiner endgültigen Form, insbesondere in seiner endgültigen geometrischen Form, auf der Außenoberfläche des Innenschafts ausgebildet wird. Dadurch wird das Verfahren zur Herstellung der Baugruppe umfassend den Innenschaft und das Führungssegment weiter vereinfacht. Insbesondere können separate Herstellungsprozesse, die gegebenenfalls einen Werkzeugwechsel erfordern oder zumindest den Wechsel zwischen Werkzeugstationen notwendig machen, wegfallen. Denn im Gegensatz zum Auflöten des Führungssegments mit anschließendem Überdrehen oder spanendem Bearbeiten, kann bei der Ausbildung des Führungssegments durch einen additiven Fertigungsprozess unmittelbar auf der Außenoberfläche des Innenschaftes die endgültige Form oder die endgültige geometrische Form des Führungssegmentes unmittelbar also direkt erzeugt werden, sodass gerade kein weiterer Bearbeitungsschritt oder keine weiteren Bearbeitungsmaßnahmen der Baugruppe umfassend das Führungssegment und den Innenschaft nach Aufbringen des Führungssegmentes mehr notwendig sind.

Eine weitere bevorzugte Ausgestaltung des Verfahrens sieht zudem vor, dass das Führungssegment mittels Laserauftragsschweißen, insbesondere von Material, welches sich mit dem Material des Innenschaftes, bevorzugt Monel verbindet, auf dem Innenschaft ausgebildet wird. Das Laserauftragsschweißen (laser metal deposition: LMD) hat im Hinblick auf die vorliegende Anwendung besondere Vorteile. Einerseits kann auch auf komplexen Oberflächen, insbesondere auch auf stark gekrümmten Oberflächen eine gleichmäßige Erzeugung eines Gegenstands gewährleistet werden. Weiter wird durch das Laserauftragsschweißen in einem Bereich unmittelbar angrenzend an das Substrat oder das Material, auf welches aufgeschweißt wird, im vorliegenden Fall also der Oberfläche des Innenschaftes ein Schmelzbad erzeugt, in dem die Materialien miteinander vermischen oder ineinander übergehen, sodass eine besonders starke und dauerhafte Verbindung zwischen dem mit dem Laserauftragsschweißen hergestellten Gegenstand und dem Untergrund oder Substrat erzeugt wird. Das aufgebrachte oder aufgetragene Material kann dabei in erster Linie anhand seiner Verbindungseigenschaften mit dem Material des Innenschaftes ausgewählt werden und zusätzlich in Hinblick auf eine Biokompatibilität gewählt werden. Die Eignung zur nachträglichen Bearbeitbarkeit kann vernachlässigt werden, da durch das erfindungsgemäße Verfahren die Möglichket eröffnet wird ohne Nachbearbeitungsschritte, insbesondere spanende Bearbeitungen zu verzichten. Dadurch kann mit dem Verfahren gemäß der vorliegenden Erfindung auch auf Messing als Material für das Führungssegment verzichtet werden.

Eine weitere Ausführungsvariante des erfindungsgemäßen Verfahrens sieht vor, dass das Führungssegment als mehrschichtiges, bevorzugt mehrteiliges, Strukturelement auf der Außenoberfläche des Innenschaftes ausgebildet wird. Durch eine mehrschichtige Ausbildung des Führungssegments kann besonders vorteilhaft eine, beispielsweise in Umfangsrichtung variierende Stärke oder Dicke des Führungssegments dadurch realisiert werden, dass in unterschiedlichen Bereichen in Umfangsrichtung unterschiedlich viele Schichten mittels des additiven Fertigungsprozesses, beispielsweise Laserauftragsschweißen, erzeugt werden. Dadurch kann besonders vorteilhaft das Führungssegment in seiner endgültigen Form direkt erzeugt werden, ohne das nachfolgende Bearbeitungsschritte oder Umformungsprozesse notwendig sind. Besonders vorteilhaft kann das Führungssegment mehrteilig ausgestaltet sein. Auch bei klassischen Führungssegmenten, die zunächst in einer Vorstufe oder einem Rohling aufgelötet und dann mittels Überdrehen in die endgültige Form gebracht werden, kann die Dicke oder Stärke des Führungssegments in manchen Bereichen bis auf 0 oder nahezu 0 reduziert werden, sodass nach dem Auflöten einer einzigen oder zusammenhängenden Vorstufe im Endeffekt zwei nahezu oder zwei tatsächlich voneinander separierte Abschnitte des Führungssegments auf der Außenoberfläche des Innenschaftes ausgebildet werden. Diese Art des Überdrehens ist eine weitere besondere Anforderung an den Herstellungsprozess bekannter Führungssegmente, weil bei dem Reduzieren der Stärke oder der Dicke des Führungssegments in Bereichen in Umfangsrichtung bis auf nahezu 0 oder bis auf 0 besondere Anforderungen an den Bearbeitungsschritt selbst sowie an die Verbindung zwischen Führungssegment und Innenschaft gewährleistet sein müssen. Durch die direkte und unmittelbare Ausbildung des Führungssegments als mehrteilige Form oder Geometrie, kann auf die entsprechenden Verfahrensschritte verzichtet werden, was ebenfalls die Voraussetzung für die Ausbildung in das Führungssegments herabsetzt oder vereinfacht.

Im Hinblick auf die Verwendung des vorangehend beschriebenen Verfahrens wird die oben genannte Aufgabe dadurch gelöst, dass das beschriebene Verfahren bei der Herstellung eines Endoskops zum Einsatz kommt oder Verwendung findet, welches einen Außenschaft mit einem Außendurchmesser von ≤4mm bevorzugt von ≤3mm besonders bevorzugt von ≤2mm, aufweist.

Grundsätzlich lassen sich vorteilhaft mit dem oben genannten Verfahren auch Führungssegmente auf Innenschäften von Endoskopen mit größerem Außendurchmesser herstellen. Wie oben bereits beschrieben kann unabhängig von den Durchmessern bei dem erfindungsgemäßen Verfahren bereits eine Vielzahl von Vorteilen gegenüber dem stand der Technik realisieren. Besonders hervorzuheben ist jedoch die Verwendung im Rahmen der Herstellung von Endoskopen mit kleinem Außendurchmesser, insbesondere mit kleinem Außendurchmesser gemäß den vorangehend genannten Grenzen. Denn wie eingangs bereits beschrieben, war die Verwendung von Führungssegmenten oder die Aufbringung von Führungssegmenten für derart kleine oder dünne Endoskope bisher nicht oder zumindest nicht mit den bekannten Methoden und Verfahren möglich.

Damit erlaubt die Verwendung erstmals in einem kompakten und wirtschaftlich sinnvollen Verfahren mit einer entsprechend hohen Zuverlässigkeit die Ausbildung oder Anordnung von Führungssegmenten auf der Außenoberfläche von Innenschäften von Endoskopen, die insgesamt nach der Montage einen Außendurchmesser von ≤4mm aufweisen.

Besonders vorteilhaft kann die Verwendung des vorangehend beschriebenen Verfahrens bei Endoskopen zum Einsatz kommen, für die im Innenschaft, auf dem das Führungssegment angeordnet oder ausgebildet wird, eine Stablinse zur Übertragung der Bildinformationen zum Einsatz kommt.

Die oben genannte Aufgabe wird im Hinblick auf ein Endoskop mit einer Lichtleitereinrichtung umfassend einen Innenschaft zur Aufnahme einer Abbildungsoptik, wobei auf dem Innenschaft ein Führungssegment zur Ausrichtung des Innenschaftes in einem Außenschaft und/oder zur Führung von Lichtleitelementen der Lichtleitereinrichtung auf der Außenoberfläche des Innenschaftes, insbesondere in einem distalen Endabschnitt des Außenschaftes angeordnet ist und wobei die Lichtleitelemente zwischen der Außenoberfläche des Innenschaftes und einer Innenoberfläche des Außenschaftes zu einem distalen Ende des Endoskops verlaufen und wobei das Endoskop ferner Befestigungsmittel zum Befestigen des Innenschaftes und der Lichtleitelemente im Außenschaft, zumindest im distalen Endabschnitt des Innenschaftes und Außenschaftes aufweist dadurch gelöst, dass das Führungssegment durch einen additiven Fertigungsprozess unmittelbar auf der Außenoberfläche des Innenschaftes angeordnet ist.

Durch die unmittelbare Ausbildung oder Aufbringung des Führungssegmentes auf der Außenoberfläche des Innenschaftes wird eine ausreichende Festigkeit oder eine ausreichende Stabilität der Verbindung zwischen Innenschaft und Führungssegment ermöglicht.

Vorteilhaft kann diese unmittelbare Verbindung mit einem additiven Fertigungsprozess erreicht werden, wodurch insgesamt Fertigungsschritte oder Herstellungsschritte gegenüber dem bekannten Stand der Technik entfallen können.

Bezüglich der Vorteile und vorteilhaften Wirkungen der erfindungsgemäßen Ausführung des Endoskops wird auch auf die vorangehende Beschreibung des entsprechend analogen erfindungsgemäßen Verfahrens verwiesen. In diesem Zusammenhang sei erwähnt, dass soweit erforderlich verfahrensmäßig offenbarte Merkmale auch als Vorrichtungsmerkmale offenbart gelten sollen und umgekehrt.

Gemäß einer ersten, besonders vorteilhaften Ausführung des Endoskops kann vorgesehen sein, dass das Endoskop ein Außenschaft aufweist, der einen Außendurchmesser von ≤4mm, bevorzugt von ≤3mm, besonders bevorzugt von ≤2mm aufweist. Durch die direkte Ausbildung des Führungssegments mittels eines additiven Verdünnungsprozesses auf der Außenoberfläche des Innenschaftes kann für derart kleine beziehungsweise dünne Endoskope erstmals zuverlässig die Anordnung eines Führungssegmentes auf dem Innenschaft ermöglicht werden und dadurch erstmals die Anordnung und Ausrichtung der Lichtleitelemente, insbesondere im distalen Endabschnitt des Endoskops, zuverlässig und reproduzierbar gewährleistet werden, sodass die Anforderungen an die Arbeitsschritte im Fertigungs- oder Montageprozess, insbesondere die Anforderungen an Montagearbeiterinnen und Montagearbeiter geringer ausfallen kann, da nicht mehr in ganz überwiegender Mehrheit die Präzision und die Erfahrung der Montagearbeiterinnen und Montagearbeiter über die Anordnung und Ausrichtung der Lichtleitelemente sowie über die Anordnung des Innenschaftes im Außenschaftes entscheidet.

Gemäß einer weiteren vorteilhaften Ausführung des Endoskops kann zudem vorgesehen sein, dass das Führungssegment aus Material, gebildet ist,welches sich mit dem Material des Innenschaftes, bevorzugt Monel verbindet, welches bevorzugt durch Laserauftragsschweißen, mit dem Material des Innenschaftes verbunden ist. Wie oben bereits ausgeführt, kann dadurch die Materialauswahl optimiert werden, da kein oder nur geringer Wert aus eine Nachbearbeitbarkeit des Materials des Führungssegments gelegt werden muss.

Eine weitere besonders bevorzugte Ausgestaltung des Endoskops kann vorsehen, dass das Führungssegment als mehrschichtiges, bevorzugt mehrteiliges Strukturelement auf der Außenoberfläche des Innenschaftes ausgebildet ist. Dadurch wird ermöglicht, dass das Führungssegment durch die Mehrschichtigkeit die, im additiven Fertigungsprozess bevorzugt abbildbar ist, die gewünschte Form, insbesondere die gewünschte Außenform und gegebenenfalls variierende Stärken oder Dicken, abhängig von der Orientierung in Umfangsrichtung, unmittelbar erzeugt oder ausgebildet wird, sodass sowohl die Führung und Ausrichtung der Lichtleitelemente, als auch die Ausrichtung des Innenschaftes im Außenschaft gewährleistet wird. Die mehrschichtige und gegebenenfalls mehrteilige Ausbildung als Strukturelement ermöglicht zudem eine genaue, gegebenenfalls exzentrische Orientierung des Innenschaftes im Außenschaft.

Eine weitere bevorzugte Ausführungsform des Endoskops sieht vor, dass dieses eine im Innenschaft angeordnete Optik aufweist, die ihrerseits wiederrum eine Stablinse zur Übertragung von Bildinformationen umfasst.

Vorteilhafte Ausführungsformen und Wirkungsweisen der vorangehend beschriebenen Erfindung werden nachfolgend anhand der Figuren, die lediglich schematisch gehalten sind, dargestellt und erläutert.

Darin zeigen:
- Fig. 1:: Eine schematische Darstellung eines distalen Endabschnitts eines Endoskops gemäß dem Stand der Technik;
- Fig. 2:: Eine schematische Darstellung eines distalen Endabschnitts eines Endoskops gemäß der vorliegenden Erfindung;
- Fig. 3:: Eine schematische Darstellung eines distalen Endabschnitts eines Innenschaftes zur Verwendung in dem erfindungsgemäßen Verfahren;
- Fig. 4:: Eine schematische Darstellung eines Ablaufdiagramms des erfindungsgemäßen Verfahrens.

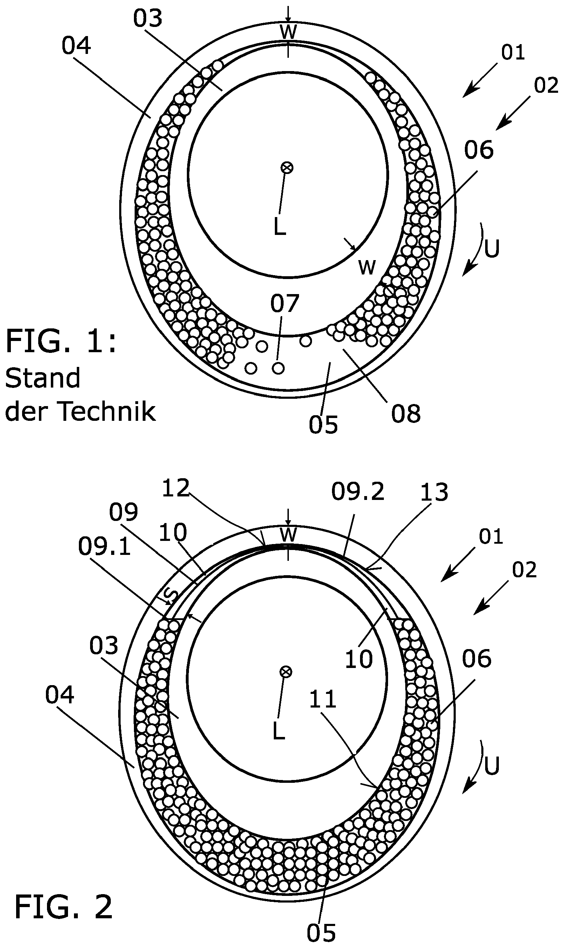

Figur 1 zeigt eine schematische Darstellung auf einen distalen Endabschnitt 01 eines Endoskops 02. Die Ansicht der Figur 1 verläuft entlang einer axialer Ausrichtung, also entlang einer gemeinsamen Längsachse L des Innenschaftes sowie des Außenschaftes. Der Innenschaft 03 ist in gewollter Weise leicht exzentrisch in dem Außenschaft 04 geführt. Die ungleichmäßigen Wandstärken W des Innenschaftes 03 sowie des Außenschaftes 04 in Umfangsrichtung U lassen erkennen, dass der distale Endabschnitt 01 gewinkelt zur Längsrichtung L ausgerichtet oder zugeschnitten ist.

Im Zwischenraum 05 zwischen Innenschaft 03 und Außenschaft 04 sind die Enden von Lichtleitelementen 06 zu erkennen, die beispielsweise als gebündelte Lichtleitfasern oder Lichtleiter ausgebildet sind. Die Lichtleitelemente 06 sind in der Darstellung der Fig. aus Gründen der Übersichtlichkeit deutlich größer/stärker dargestellt als in der Realität. In der Figur 1 ist zu erkennen, dass die Enden der Lichtelemente 06 im Bereich des distalen Endabschnitts 01 in Umfangsrichtung U in einer unerwünschten oder wenig erwünschten Weise ungleichmäßig verteilt sind und insbesondere in einem Bereich 07 des Zwischenraums 05 nur sehr vereinzelt Lichtleitelemente 06 angeordnet sind oder bereichsweise überhaupt keine Lichtleitelemente 06 angeordnet sind. Dies führt zu einer unerwünschten oder nicht gewollten Beleuchtungssituation. In dem Bereich 07 wird im Rahmen der Montage der Zwischenraum 05 beispielsweise mit Befestigungsmittel 08 in Form von Haftvermittler oder Klebstoff ausgefüllt, der auch ansonsten die Befestigung oder Sicherung des Innenschafts im Außenschaft sowie die Sicherung und Befestigung der Lichtleitelemente 06 im distalen Endabschnitt 01 sicherstellt.

Die Figur 1 zeigt ein distalen Endabschnitt 01 eines Endoskops mit einem kleinen Durchmesser, insbesondere mit einem kleinen Außendurchmesser des Außenschafts 04, der beispielsweise ≤4mm oder sogar ≤3mm ausfällt. Bei derart kleinen oder dünnen Endoskopen und dazugehörigen distalen Endabschnitten 01 war die Situation, wie sie in Figur 1 beispielhaft dargestellt ist, in einem Teil der Montagefälle unvermeidlich, da es bis dato nicht oder zumindest nicht reproduzierbar oder wirtschaftlich möglich war für Endoskope diesen Dimensionen ein Führungssegment auf dem Innenschaft 03 anzuordnen oder auszubilden, welches die Positionierung des Innenschafts 03 im Außenschaft 04 und auch die Anordnung und Ausrichtung der Lichtleitelemente 06 verbessert.

Genau diese Ausbildung oder Anordnung eines Führungssegments 09 ist, wie in Figur 2 schematisch dargestellt, gemäß der vorliegenden Erfindung, also mit dem erfindungsgemäßen Endoskop und dem erfindungsgemäßen Verfahren jetzt nicht nur grundsätzlich in vorteilhafter Weise sondern besonders bevorzugt auch für Endoskope mit kleine, Außendurchmesser möglich.

Die Figur 2 zeigt, insbesondere im Vergleich mit der Figur 1 eine gleich ausgerichtete Ansicht eines distalen Endabschnitts 01 eines Endoskops 02, bei dem in einem Teil des Zwischenraums 05 zwischen Innenschaft 03 und Außenschaft 04 ein erfindungsgemäßes Führungssegment 09 angeordnet ist. Dieses sorgt, wie leicht zu erkennen ist, für die gewünschte Anordnung und Ausrichtung der Lichtleitelemente 06 sowie auch für die beabsichtigte Ausrichtung des Innenschaftes 03 im Außenschaft 04, insbesondere im distalen Endabschnitt 01.

Das Führungssegment 09 ist dabei als mehrschichtiges und mehrteiliges Strukturelement ausgebildet, was durch die Schichten 10 und die einzelnen Teile 09.1 und 09.2 angedeutet wird. Die einzelnen Schichten 10 werden in einem additiven Fertigungsprozess, beispielsweise in einem Laserauftragsschweißverfahren erzeugt, wobei das Führungssegment 09 derart erzeugt wird, dass es in seiner endgültigen Form und insbesondere ohne weitere Nachbearbeitungsschritte unmittelbar auf der Außenoberfläche 11 des Innenschaftes 03 ausgebildet und/oder angeordnet wird. Damit kann insbesondere für Endoskope 02 mit kleinem Durchmesser ein spanender Bearbeitungsschritt, bevorzugt zur Ausbildung der unterschiedlichen Stärken S des Führungssegments 09 in Umfangsrichtung U unterbleiben oder wegfallen. Es ist deutlich erkennbar, dass die Verteilung der Lichtelemente 06 gegenüber der Figur 1 verbessert ist und das auch die Ausrichtung des Innenschaftes 03 im Außenschaft 04 einfacher und reproduzierbarer erfolgen kann, wenn beim Einführen des Innenschaftes 03 in den Außenschaft 04 die radial äußere Oberfläche 12 des Führungssegments 09 an der Innenoberfläche 13 des Außenschafts 04 zur Anlage kommt und/oder entlang geführt wird, bis das distale Ende des Innenschaftes 03 zusammen mit dem Lichtleitelementen 06 das distale Ende des Außenschaftes 04 erreicht hat.

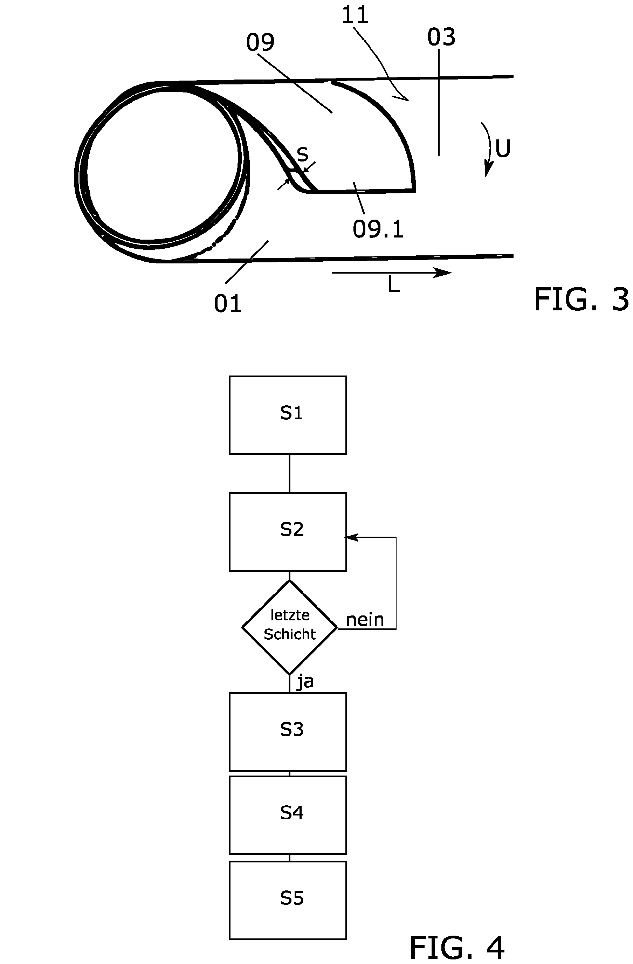

Die Figur 3 zeigt eine beispielhafte Ausgestaltung eines Teils 09.1 eines mehrteilig als Strukturelement ausgebildeten Führungssegments 09 auf dem distalen Endabschnitt 01 eines Innenschaftes 03. In der perspektivischen Ansicht der Figur 3 ist dabei einerseits zu erkennen, dass das Führungssegment 09 in Umfangsrichtung U eine variierende Wandstärke oder Dicke S aufweist und dass das Führungssegment 09 in axialer Richtung oder in Richtung der Längsachse L unterschiedliche Abschnitte mit sowohl geraden als auch gekrümmten Konturen aufweist. Eine derartige endgültige Form des Führungssegments 09 kann in besonders vorteilhafter Weise durch einen additiven Fertigungsprozess unmittelbar auf der Außenoberfläche 11 des Innenschaftes 03, beispielsweise durch Laserauftragsschweißen auf den Außenschaft 03 ausgebildet werden. Eine bevorzugte Mehrschichtigkeit des Führungssegments 09 ist aus Gründen der Übersichtlichkeit in der Darstellung der Figur 3 nicht gezeigt.

Die Figur 4 zeigt einen beispielhaften und stark vereinfachten Verfahrensablauf eines erfindungsgemäßen Verfahrens. Grundsätzlich bekannte Einzelschritte im Rahmen der Herstellung oder Fertigung eines Endoskops, insbesondere im Rahmen der Herstellung eines Endoskops mit kleinem Außendurchmesser, bevorzugt von ≤4mm, sind aus Gründen der Übersichtlichkeit nicht dargestellt worden, sind jedoch vom Gesamtprozess als umfasst anzusehen.

In dem erfindungsgemäß relevanten Abschnitt des gesamten Herstellungsprozesses oder Herstellungsverfahren wird in einem ersten Verfahrensschritt S1 ein Innenschaft zur Aufnahme einer Abbildungsoptik bereitgestellt. In einem nachfolgenden Verfahrensschritt S2 wird in Abschnitten oder Bereichen des distalen Endabschnitts des Innenschaftes eine erste Lage in einem oder mehreren voneinander getrennten Bereichen oder Abschnitten des Endabschnitts des Innenrohrs mit einem additiven Fertigungsprozess, beispielsweise mit Laserauftragsschweißen ausgebildet. Dieser Verfahrensschritt S2 wird mit dem Auftrag von weiteren Schichten, jeweils auf der vorangehend ausgebildeten Schicht und/oder auf der Außenoberfläche des Innenschaftes, solange ausgeführt, bis das Führungssegment vollständig und in seiner endgültigen Form auf dem Innenschaft ausgebildet ist und folglich die letzte Schicht aufgetragen ist. Dabei erfolgt in besonders vorteilhafter Weise eine unmittelbare Aufbringung des Führungssegments auf dem Innenschaft, beispielsweise unter Ausbildung einer Schweißverbindung zwischen dem Material des Innenschaftes einerseits und der ersten Lage oder der ersten Schicht des Führungssegments andererseits.

Nach der rekursiven Ausbildung des Führungssegments auf dem Innenschaft im Rahmen des Verfahrensschritt S2 erfolgt im Verfahrensschritt S3 die Anordnung der Lichtleitelemente der Lichtleitereinrichtung auf der Außenoberfläche des Innenschaftes sowie in Anlage an das Führungssegment. In einem nachfolgenden Verfahrensschritt wird der Innenschaft samt Lichtleitelementen in de Außenschaft eingeführt, wobei die radial äußere Oberfläche des Führungssegments an der Innenoberfläche des Außenschafts zur Anlage kommt. Dies soll im beispielhaften Verfahrensschritt S4 erfolgen.

Im nachfolgenden Verfahrensschritt S5 kann, beispielsweise unter Verwendung eines Haftvermittlers oder Klebstoffs eine Befestigung des Innenschaftes und der Lichtleitelemente im Außenschaft, zumindest im distalen Endabschnitt des Innenschaftes und Außenschaftes erfolgen.

### Bezugszeichenliste:

- 01: distalen Endabschnitt
- 02: Endoskop
- 03: Innenschaft
- 04: Außenschaft
- 05: Zwischenraum
- 06: Lichtleitelement
- 07: Bereich
- 08: Befestigungsmittel
- 09: Führungssegment
- 09.1: erster Teil
- 09.2: zweiter Teil
- 10: Schicht
- 11: Außenoberfläche des Innenschaftes
- 12: Außenoberfläche des Führungssegments
- 13: Innenoberfläche des Außenschaftes

- W: Wandstärke
- S: Dicke des Führungssegments (radial)
- U: Umfangsrichtung
- L: Längsrichtung (Innenschaft und Außenschaft)

- S1 - S5: Verfahrensschritte

## Patentansprüche

1. Verfahren zur Herstellung eines Endoskops (02) mit einer Lichtleitereinrichtung umfassend die Verfahrensschritte:
- Bereitstellen eines Innenschaftes (03) zur Aufnahme einer Abbildungsoptik (S1);
- Anordnen eines Führungssegmentes (09) zur Ausrichtung des Innenschaftes (03) in einem Außenschaft (04) und/oder zur Führung von Lichtleitelementen (06) der Lichtleitereinrichtung auf der Außenoberfläche (11) des Innenschaftes (03), insbesondere in einem distalen Endabschnitt (01) des Außenschaftes (04);
- Anordnen der Lichtleitelemente (06) auf der Außenoberfläche (11) des Innenschaftes (03; S3);
- Einführen des Innenschaftes (03) samt Lichtleitelementen (06) in den Außenschaft (04) unter Anlage einer radial äußeren Oberfläche (12) des Führungssegments (09) an einer Innenoberfläche (13) des Außenschafts (04; S4);
- Befestigen des Innenschaftes (03) und der Lichtleitelemente (06) im Außenschaft (04), zumindest im distalen Endabschnitt (01) des Innenschaftes (03) und Außenschaftes (04; S5),
**dadurch gekennzeichnet,**
**dass** das Führungssegment (09) durch einen additiven Fertigungsprozess unmittelbar auf der Außenoberfläche (11) des Innenschaftes (03) ausgebildet wird (S2).

2. Verfahren nach Anspruch 1,
**dadurch gekennzeichnet,**
**dass** das Führungssegment (09) in seiner endgültigen Form auf der Außenoberfläche des Innenschaftes (11) ausgebildet wird.

3. Verfahren nach Anspruch 1 oder 2,
**dadurch gekennzeichnet,**
**dass** das Führungssegment (09) mittels Laserauftragsschweißen (laser metal deposition, "LMD"), insbesondere von Material, welches sich mit dem Material des Innenschaftes, bevorzugt Monel, verbindet, auf dem Innenschaft (03) ausgebildet wird.

4. Verfahren nach einem der Ansprüche 1 bis 3,
**dadurch gekennzeichnet,**
**dass** das Führungssegment (09) als mehrschichtiges, bevorzugt mehrteiliges, Strukturelement auf der Außenoberfläche des Innenschaftes (11) ausgebildet wird.

5. Verwendung des Verfahrens nach einem der Ansprüche 1 bis 4 zur Herstellung eines Endoskops (02),
**dadurch gekennzeichnet,**
**dass** das Endoskop (02) einen Außenschaft (04) mit einem Außendurchmesser von ≤4mm, bevorzugt mit ≤3mm, besonders bevorzugt mit ≤2mm aufweist.

6. Endoskop (02) mit einer Lichtleitereinrichtung umfassend eines Innenschaft (03) zur Aufnahme einer Abbildungsoptik, wobei auf dem Innenschaft (03) ein Führungssegment (09) zur Ausrichtung des Innenschaftes (03) in einem Außenschaft (04) und/oder zur Führung von Lichtleitelementen (06) der Lichtleitereinrichtung auf der Außenoberfläche des Innenschaftes (11), insbesondere in einem distalen Endabschnitt (01) des Außenschaftes (04) angeordnet ist und wobei die Lichtleitelemente (06) zwischen der Außenoberfläche des Innenschaftes (11) und einer Innenoberfläche des Außenschaftes (13) zu einem distalen Ende des Endoskopes (02) verlaufen und wobei das Endoskop (02) fernen Befestigungsmittel (08) zum Befestigen des Innenschaftes (03) und der Lichtleitelemente (06) im Außenschaft (04), zumindest im distalen Endabschnitt des Innenschaftes (03) und Außenschaftes (04), aufweist,
**dadurch gekennzeichnet,**
**dass** das Führungssegment (09) durch einen additiven Fertigungsprozess unmittelbar auf der Außenoberfläche des Innenschaftes (11) angeordnet ist.

7. Endoskop (02) nach Anspruch 6,
**dadurch gekennzeichnet,**
**dass** der Außenschaft (04) des Endoskops (02) einen Außendurchmesser von ≤4mm, bevorzugt mit ≤3mm, besonders bevorzugt mit ≤2mm, aufweist.

8. Endoskop (02) nach Anspruch 6 oder 7,
**dadurch gekennzeichnet,**
**dass** das Führungssegment (09) aus Material gebildet ist, welches, bevorzugt durch Laserauftragsschweißen, mit dem Material des Innenschaftes (03), bevorzugt Monel, verbunden ist.

9. Endoskop (02) nach einem der Ansprüche 6 bis 8,
**dadurch gekennzeichnet,**
**dass** das Führungssegment (09) als mehrschichtiges, bevorzugt mehrteiliges, Strukturelement auf der Außenoberfläche des Innenschaftes (11) ausgebildet ist.

10. Endoskop (02) nach einem der Ansprüche 6 bis 9,
**dadurch gekennzeichnet,**
**dass** eine im Innenschaft (03) angeordnete Optik des Endoskops (02) eine Stablinse aufweist.
